Europäisches Patentamt

European Patent Office

Office européen des brevets

(19)

(11) Publication number: **0 079 793**
**B1**

(12) **EUROPEAN PATENT SPECIFICATION**

(45) Date of publication of patent specification: **10.09.86**

(21) Application number: **82306077.7**

(22) Date of filing: **15.11.82**

(51) Int. Cl.⁴: **C 12 Q 1/40,** C 12 N 9/00,
A 61 K 37/64

(54) Amylase inhibitor and preparation thereof.

(30) Priority: **16.11.81 JP 182316/81**

(43) Date of publication of application:
**25.05.83 Bulletin 83/21**

(45) Publication of the grant of the patent:
**10.09.86 Bulletin 86/37**

(84) Designated Contracting States:
**DE FR GB IT NL**

(56) References cited:
**US-A-3 944 537**
**US-A-3 950 319**

**BIOLOGICAL ABSTRACTS, vol. 61, 1976,**
**abstract no. 60038, Philadelphia, Pa., USA M.D.**
**O'DONNELL et al.: "Purification and properties**
**of an alpha amylase inhibitor from wheat"**

**BIOLOGICAL ABSTRACTS, vol. 63, no. 12, 1977,**
**abstract no. 68506, Philadelphia, Pa., USA R.R.**
**DEPONTE et al.: "Albumin alpha-amylase**
**inhibitor families from wheat four"**

(73) Proprietor: **FUJIREBIO KABUSHIKI KAISHA also**
**trading as FUJIREBIO INC.**
**6-7, Shimoochiai 4-chome Shinjuku-ku**
**Tokyo 161 (JP)**

(72) Inventor: **Sugiyama, Masami**
**9-19, Motoyokoyamamachi 2-chome**
**Hachioji-shi Tokyo (JP)**
Inventor: **Mashara, Yasushi**
**310, 4-4, Nagayama 3-chome**
**Tama-shi Tokyo (JP)**

(74) Representative: **Silverman, Warren et al**
**HASELTINE LAKE & CO. Hazlitt House 28**
**Southampton Buildings Chancery Lane**
**London WC2A 1AT (GB)**

(56) References cited:

BIOLOGICAL ABSTRACTS, vol. 66, 1978,
abstract no. 20977, Philadelphia, Pa., USA J.R.
WARCHALEWSKI- "Isolation and purification of
native alpha-amylase inhibitors from malted
winter wheat"

BIOLOGICAL ABSTRACTS, vol. 66, 1978,
abstract no. 20978, Philadelphia, Pa., USA J.R.
WARCHALEWSKI: "Isolation and purification of
native alpha-amylase inhibitors from winter
wheat"

BIOLOGICAL ABSTRACTS, vol. 72, 1981,
abstract no. 23636, Philadelphia, Pa., USA C.M.
O'CONNOR et al.: "Isolation and
characterization of 4 inhibitors from wheat
flour which display differential inhibition
specificities for human salivary and human
pancreatic alpha-amylases"

## Description

This invention relates to an amylase inhibitor, to use in the detection of amylase isoenzymes and to a process for the preparation thereof.

Various amylase inhibitors are known. In general, however, amylase inhibitors which specifically inhibit salivary amylase, inhibit pancreatic amylase too. For instance, it is known that an amylase inhibitor is present in wheat (M.D. O'Donnel et al., Biochim. Biophys. Acta, Vol. 422, pp 159—169 (1976)). This amylase inhibitor inhibits not only salivary amylase, but also pancreatic amylase.

It has now been found that a previously unreported amylase inhibitor, which we have named Sain, and which exhibits a ratio of the inhibition rate against pancreatic amylase to the inhibition rate against salivary amylase of less than 0.1, can be obtained from hard wheat and durum wheat by dye ligand affinity chromatography.

Thus according to one aspect of the invention, there is provided an amylase inhibitor having an inhibitory activity on human pancreatic amylase which is less than 0.1 of its inhibitory activity on human salivary amylase, which inhibitor has:

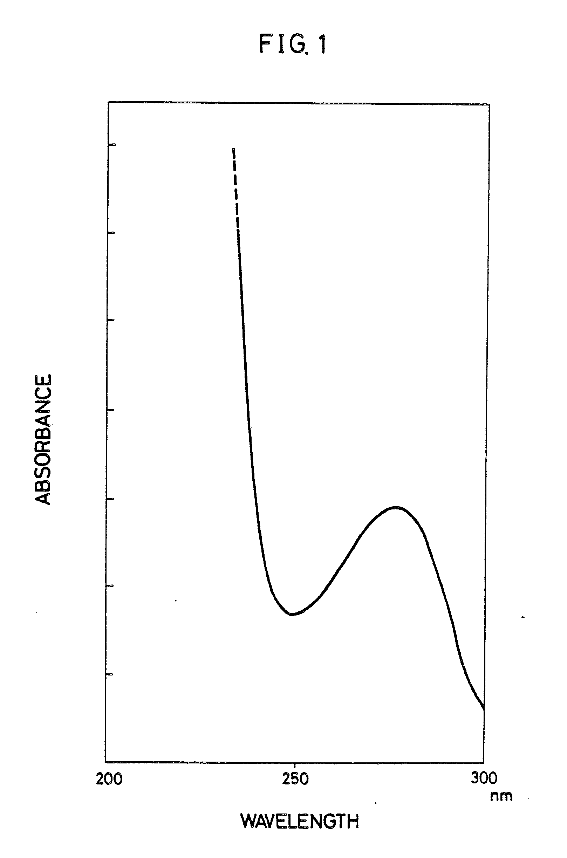

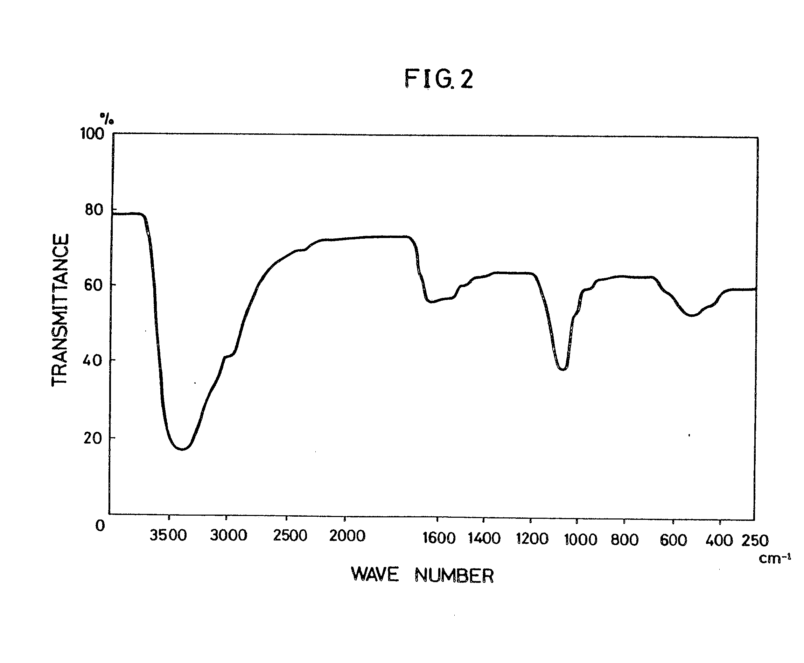

(a) a molecular weight in the range of from 23,000 to 24,000 determined by gel filtration; (b) an ultraviolet absorption spectrum as shown in Figure 1 of the accompanying drawings for a 0.22% by weight aqueous solution of the amylase inhibitor; (c) an infrared absorption spectrum, when pelleted with potassium bromide, as shown in Figure 2 of the accompanying drawings; (d) an isoelectric point of 5.0; (e) zero carbohydrate content and (f) the following amino acid composition:

| Tyr | 17 units | Ser | 12 units |
|-----|----------|-----|----------|
| Leu | 6 units | Thr | 6 units |
| Ile | 4 units | Asp | 17 units |
| Met | 10 units | Arg | 9 units |
| Val | 24 units | His | 2 units |
| Ala | 24 units | Lys | 9 units |
| Gly | 22 units | Phe | 5 units |
| Pro | 11 units | 1/2 Cys | 14 units |
| Glu | 22 units | | |

This invention also provides in a second aspect a process for the production of an amylase inhibitor according to the first aspect of the invention, which comprises soaking hard wheat or durum wheat in water or an aqueous/organic solvent, subjecting the extract thereby obtained to dye ligand affinity chromatography, eluting the amylase inhibitor from the immobilised dye employed and isolating the inhibitor from the eluate.

Since the amylase inhibitor Sain specifically inhibits salivary amylase, it may be used to detect and measure amylase isoenzymes as an alternative to conventional electrophoresis procedures. Hence, in a third aspect, this invention provides a method of quantitatively determining the presence of amylase isoenzyme in a fluid, which comprises inhibiting salivary amylase activity in the fluid by addition thereto of an amylase inhibitor according to the first aspect of the invention and then carrying out the required amylase isoenzyme determination.

To produce Sain, grains of the above wheat are first milled and the wheat flour is mixed with water or an aqueous solution such as a buffer solution in an amount of 5 to 20 times the volume of the wheat flour. The mixture obtained is stirred for from 30 minutes to 2 hours, and then filtered or centrifuged to obtain an extract of the amylase inhibitor. During extraction, the mixture may be heated to a temperature not exceeding the temperature at which the starch content of the wheat becomes gelatinised.

The dye ligand affinity chromatography may be carried out using the reaction product of a reactive dye and a carrier having hydroxyl groups or amino groups. Such carriers include Sepharose® 4B and Sepharose® CL-6B (manufactured by Pharmacia AB), Sephadex® G-200 (manufactured by Pharmacia AB), Biogel-P (manufactured by Bio Rad Co.), and cellulose.

The reactive dye will generally be a derivative of cyanuric chloride, for example reactive red or reactive blue.

The reaction of the reactive dye with the carrier may be carried out in the following manner. The carrier is suspended in water, and the suspension is made alkaline. Then the reactive dye is added to the alkaline suspension, and the suspension is warmed and subjected to gentle stirring. This results in chloride of the cyanuric chloride moeity reacting with the hydroxyl groups or the amino groups of the carrier, to form a covalent bond between the reactive dye and the carrier.

The amount of the reactive dye used is usually from 0.1 to 1 gram per 100 ml of the carrier in its swelled form. The alkali added to the suspension may be sodium hydroxide, potassium hydroxide or sodium carbonate, and the pH of the suspension is preferably adjusted to from 9 to 11. The temperature rise when carrying out the reaction may be accelerated by external heating. After the reaction, the carrier on which the reactive dye is immobilized is filtered off, and washed several times with a suitable solvent, for example water or acetone.

The thus produced immobilized reactive dye is packed in a column, and a solution containing the amylase inhibitor Sain is passed through the column. The adsorbed material on the immobilized reactive dye is then eluted by means of a developing agent. The amount of solution fed to the column is preferably such that from 5 to 10 g of the amylase inhibitor are adsorbed per litre of the immobilized reactive dye. A phosphate buffer solution-acetone gradient or one of various gradients which can be produced with a pyrophosphate buffer solution and a polar solvent such as ethanol, methanol or acetone may be employed as the developing agent. The eluate obtained is fractionated and the fractions analysed by using for example ultraviolet absorption or the protein concentration method according to Folin-Lowry. The fractions containing the amylase inhibitor Sain are then collected. After the elution, the immobilized reactive dye may be regenerated using any convenient method.

While it is known that enzymes in general can be purified by dye ligand affinity chromatography, it has never been proposed before to isolate an enzyme inhibitor, such as the amylase inhibitor Sain, by using dye ligand affinity chromatography. It has now been found that at least the amylase inhibitor can be purified by this procedure.

When the amylase inhibitor Sain is isolated from a wheat flour extract, other purification methods may also be employed in conjunction with the dye ligand affinity chromatography procedure. Such purification methods include ion-exchange chromatography, gel filtration, fractional precipitation using alcohol, fractional precipitation using ammonium sulphate, and dialysis which are general purification methods for proteins.

The amylase inhibitor Sain may be separated from other amylase inhibitors by using dye ligand affinity chromatography. At that time, most impurities are also removed from the amylase inhibitor Sain. The amylase inhibitor Sain may be obtained in pure form from the aqueous extract of wheat by successively carrying out two dye ligand affinity chromatography operations or by subjecting the aqueous extract once to the dye affinity chromatography procedure and then subjecting the eluate obtained to ion-exchange chromatography.

The amylase inhibitor Sain obtained according to the method of this invention has the following properties.

1. Molecular weight

The molecular weight of the amylase inhibitor obtained by the method of this invention has been determined by gel filtration using for example Sephadex G-100 to lie in the range of from 23,000 to 24,000. Since the molecular weight determined by means of electrophoresis using sodium dodecyl sulphate and polyacrylamide gel is in the range of from 11,000 to 13,000, the amylase inhibitor Sain is believed to be a dimer.

2. Ultraviolet absorption spectrum

The ultraviolet absorption spectrum of a 0.22% by weight aqueous solution of the amylase inhibitor was measured, and is shown in Figure 1 of the accompanying drawings.

3. Infrared absorption spectrum

The infrared absorption spectrum of the amylase inhibitor when pelleted with potassium bromide was measured, and is shown in Figure 2 of the accompanying drawings.

4. Isoelectric point

The isoelectric point of the amylase inhibitor measured by using Bio Lyte (manufactured by Bio Rad Co.) is 5.0.

5. Content of carbohydrate

The carbohydrate content of the amylase inhibitor measured by means of the phenol-sulphuric acid method is zero.

6. Amino acid composition

The amylase inhibitor was hydrolysed with 6N hydrochloric acid at 110°C for 22 hours, and the number of units of each amino acid in the inhibitor was determined by analysis of the hydrolsate. The amino acid composition (in amino acid units) estimated from the amino acid content and the molecular weight of the inhibitor is as follows:

| Tyr | 17 units | Ser | 12 units |
|-----|----------|-----|----------|
| Leu | 6 units | Thr | 6 units |
| Ile | 4 units | Asp | 17 units |
| Met | 10 units | Arg | 9 units |
| Val | 24 units | His | 2 units |
| Ala | 24 units | Lys | 9 units |
| Gly | 22 units | Phe | 5 units |
| Pro | 11 units | 1/2 Cys | 14 units |
| Glu | 22 units | | |

7. Inhibition of amylase

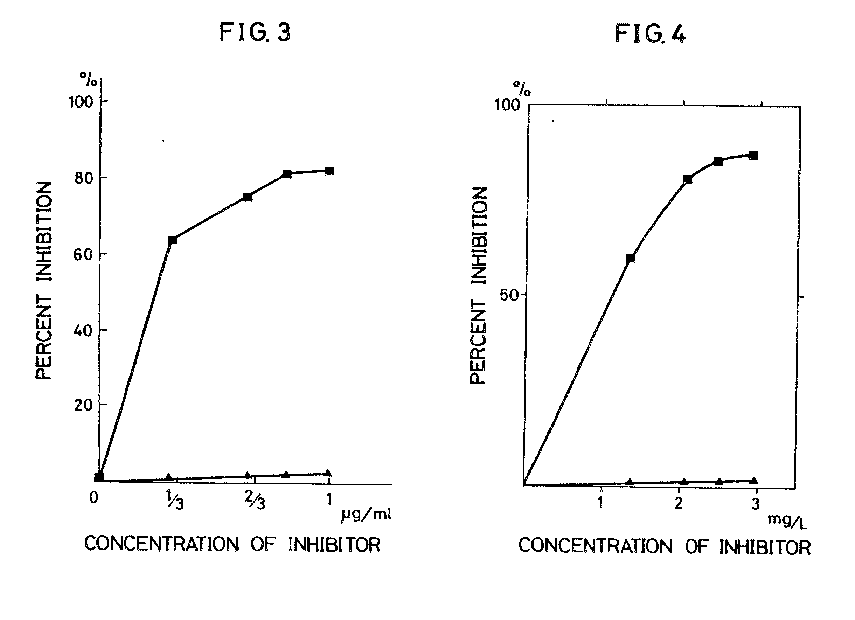

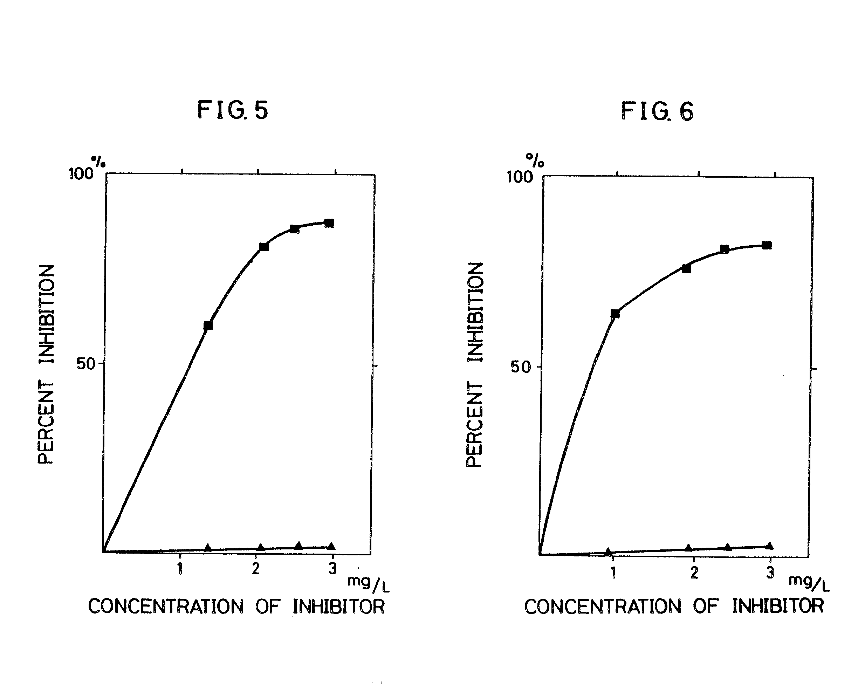

Using Phadebas Blue starch (Pharmacia Ab) as substrate, the amylase DS test (Beckman Instrument Co.) was carried out to determine the effects of the amylase inhibitor on human salivary amylase and human pancreatic amylase which are respectively represented by squares and triangles in the plots shown in Figure 3 of the accompanying drawings. Inhibition tests were also carried out using Blue starch (Pharmacia AB), maltotetraose and p-nitrophenylmaltoheptaoside as substrate, and the results obtained are shown in Figures 4 to 6 respectively of the accompanying drawings.

As can be seen from these drawings, the amylase inhibitor specifically inhibits salivary amylase and does not inhibit pancreatic amylase.

8. Inhibition type

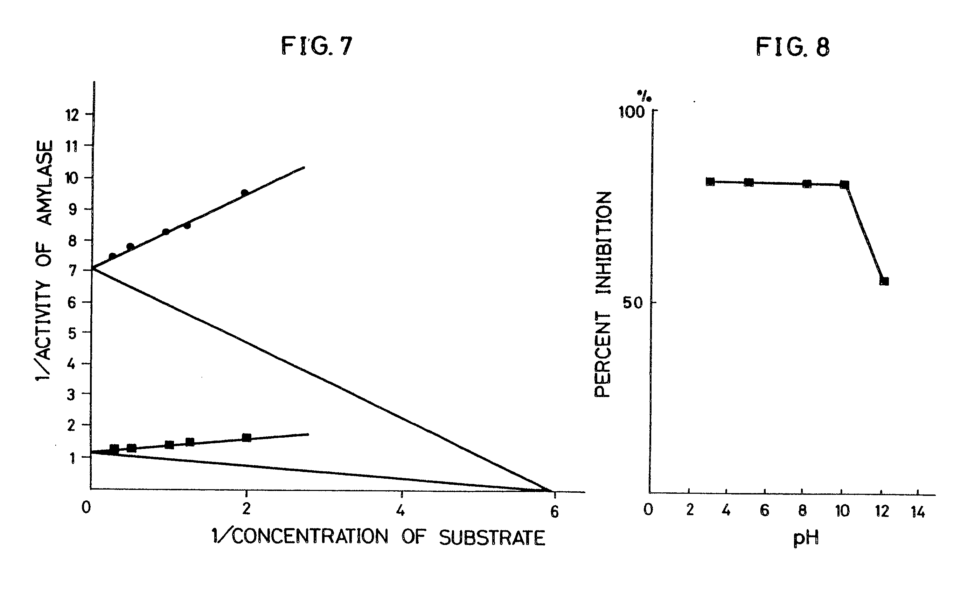

The inhibition type of the amylase inhibitor of the invention was measured using Blue starch (Pharmacia AB) as substrate and human salivary amylase, and the results obtained are shown in Figure 7 of the accompanying drawings. In Figure 7, a solid circle indicates that the amylase inhibitor is present, and a square indicates that the amylase inhibitor is absent.

As can be seen from the figure, the amylase inhibitor of the present invention belongs to the non-competitive type. The inhibition constant ($K_i$) was $8.4 \times 10^{-9}$M.

9. pH stability

Aqueous solution each containing 38 µg/ml of the amylase inhibitor which had been previously adjusted to different pH values were heated at 37°C for 30 minutes, and thereafter the inhibitory activities of the solutions on human salivary amylase were measured. The results obtained are shown in Figure 8 of the accompanying drawings and show that the amylase inhibitor is effective at pH values up to at least 12.

10. Thermal stability

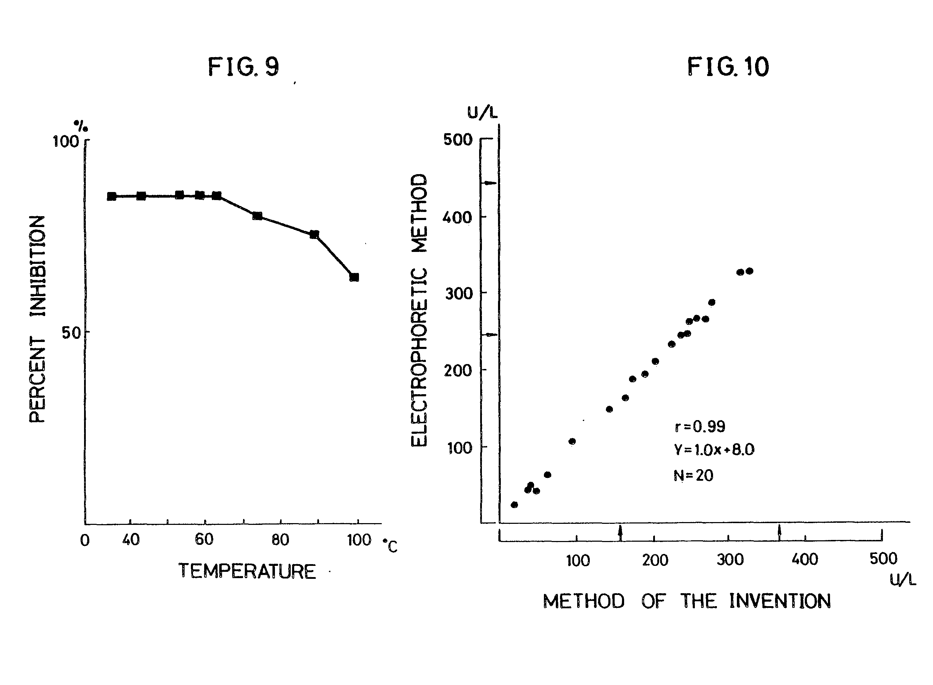

Aqueous solutions containing 38 µg/ml of the amylase inhibitor at a pH of 7.0 were heated at different temperatures for 30 minutes, and the residual inhibitory activity of each solution on human salivary amylase was measured. The results obtained are shown in Figure 9 of the accompanying drawings. It can be seen that activity is retained in full up to about 60°C and then gradually decreases.

A comparison of the above-mentioned properties of the amylase inhibitor of this invention with the properties of known amylase inhibitors shows that the present amylase inhibitor is fundamentally different from known amylase inhibitors. For example, every amylase inhibitor hitherto obtained from wheat inhibits both salivary amylase and pancreatic amylase. Furthermore, amylase inhibitors hitherto obtained from wheat have molecular weights in the range of 13,000 to 20,000. In contradistinction, the amylase inhibitor of this invention does not inhibit pancreatic amylase and has a molecular weight in the range of from 23,000 to 24,000.

Samples of human urine containing various concentrations of amylase isoenzymes were measured both using the amylase inhibitor Sain obtained in Example 1 which follows and by the conventional electrophoretic method and the activities (U/L) obtained are compared in Figure 10 of the accompanying drawings, wherein r denotes the correlation coefficient, Y denotes primary regression and N denotes the number of samples. As can be seen from Figure 10, the data obtained in the method using the amylase inhibitor Sain correlated with those obtained in the conventional method.

In carrying out the various experiments whose results are shown in the accompanying drawings, the following amylase activity and amylase inhibitory activity assay methods were used.

To determine amylase activity, one tablet of Blue starch (manufactured by Pharmacia AB) is added to 100 µl of any amylase solution, and incubated at 37°C for 15 minutes. The absorbancy of the solution at 620 nm is measured, and the amylase activity is expressed in international units.

To determine amylase inhibitor activity, human blood serum or urine is used as an amylase solution. 100 µl of the amylase solution are added to 100 µl of an anylase inhibitor solution, and the mixture is incubated at 37°C for 5 minutes. One tablet of Blue starch (manufactured by Pharmacia AB) is added to the mixture, and the solution is incubated at 37°C for 15 minutes. The absorbancy of the solution at 620 nm is measured, and one unit of amylase inhibitory activity is defined as the amount of inhibitory activity to inactivate 50% of the salivary amylase in the amylase solution.

The following Examples illustrate this invention:

Example 1

In a preliminary step, packing for a column for use in dye ligand affinity chromatography was produced. For this purpose, 100 ml of Sepharose® 413 (manufactured by Pharmacia AB) were suspended in 200 ml of water. 4 g of sodium carbonate were added to the suspension, to make the suspension alkaline. One gram of reactive red (made by Sigma Chemicals Co.) was added to the alkaline suspension, and allowed to react at 40°C for 40 hours. After the reaction, the suspension was filtered, and the residue was washed 10 times with 500 ml of water, and then 2 times with 500 ml of acetone. The immobilized reactive red thus obtained was stored at 4°C, and it was found that the immobilized reactive red was stable for more than several months.

1000 g of wheat flour produced from hard wheat (manufactured by Nisshin Flour Milling Co., Ltd.) were suspended in 8 volumes of water, and the suspension was stirred at ambient temperature for 1 hour. The wheat flour was filtered off, and washed with a small amount of water which was combined with the filtrate to obtain 6 litres of a crude extract.

3 kg of ammonium sulphate were dissolved in the crude extract, which was allowed to stand for 1 hour at ambient temperature. The precipitate which formed was collected by centrifuging, and dialysed for one day against distilled water. The dialysate was then lyophilised.

120 ml of the immobilized reactive red obtained as aforesaid were packed in a column. One gram of the lyophilised dialysate was dissolved in 100 ml of 10 mM phosphate buffer solution having a pH of 6.0, and the solution was passed downwardly through the above column. The adsorbates on the immobilized reactive red were developed by means of gradient elution by first using 10 mM phosphate buffer solution having a pH of 6.0 and then using acetone whose concentration in the column was steadily varied from 0 to 40 v/v%. The effluent from the column was fractionated into 9 ml portions by using a fraction collector. Inhibitory activity on salivary amylase and on pancreatic amylase were measured for each fraction and the fractions which specifically inhibited salivary amylase were collected and combined.

The combined fractions were dialysed for one day against water, and the residue obtained was concentrated to one tenth of its original volume. The concentrate was diluted with tris-HCl buffer solution having a pH of 7.4, and the diluted concentrate was passed through the column containing 20 ml of the ion-exchange resin DE-52 (made by Whatman Co.) at a rate of 3 ml/hr. The eluate was fractionated into 2.1 ml portions by using a fraction collector. Ultraviolet absorption of each fraction at 280 nm was carried out, and those fractions which indicated absorption, were also measured for amylase inhibitory activity.

The fractions having amylase inhibitory activity were collected, and dialysed for one day against distilled water. The residue obtained was lyophilised to obtain 0.1 g of the amylase inhibitor Sain in the form of white powder.

Example 2

100 g of durum wheat flour were treated by the procedure employed in Example 1, with the only difference being that the ion-exchange chromatography step was replaced by dye ligand affinity chromatography and 0.11 g of a white powder were obtained. Amylase inhibitory activity, molecular weight, ultraviolet absorption and isoelectric point of this white powder were measured, and it was confirmed that the white powder was substantially identical with the white powder obtained in Example 1.

**Claims**

1. An amylase inhibitor having an inhibitory activity on human pancreatic amylase which is less than 0.1 of its inhibitory activity on human salivary amylase, which inhibitor has:

(a) a molecular weight in the range of from 23,000 to 24,000 determined by gel filtration; (b) an ultraviolet absorption spectrum as shown in Figure 1 of the accompanying drawings for a 0.22% by weight aqueous solution of the amylase inhibitor; (c) an infrared absorption spectrum, when pelleted with potassium bromide, as shown in Figure 2 of the accompanying drawings; (d) an isoelectric point of 5.0; (e) zero carbohydrate content and (f) the following amino acid composition:

| | | | |
|---|---|---|---|
| Tyr | 17 units | Ser | 12 units |
| Leu | 6 units | Thr | 6 units |
| Ile | 4 units | Asp | 17 units |
| Met | 10 units | Arg | 9 units |
| Val | 24 units | His | 2 units |
| Ala | 24 units | Lys | 9 units |
| Gly | 22 units | Phe | 5 units |
| Pro | 11 units | 1/2 Cys | 14 units |
| Glu | 22 units | | |

2. A process for the production of an amylase inhibitor according to claim 1, which comprises soaking hard wheat or durum wheat in water or an aqueous/organic solvent, subjecting the extract thereby obtained to dye ligand affinity chromatography, eluting the amylase inhibitor from the immobilised dye employed and isolating the inhibitor from the eluate.

3. A process as claimed in claim 2, wherein the dye is a cyanuric chloride derivative.

4. A process as claimed in claim 3, wherein the dye is reactive red or reactive blue.

5. A process as claimed in any one of claims 2 to 4, wherein the inhibitor is purified by repetition of the dye ligand affinity chromatography or by gel filtration, fractional precipitation using alcohol or ammonium sulphate or dialysis.

6. A method of quantitatively determining the presence of amylase isoenzyme in a fluid, which comprises inhibiting salivary amylase activity in the fluid by addition thereto of an amylase inhibitor as claimed in Claim 1 and then carrying out the required amylase isoenzyme determination.

**Patentansprüche**

1. Amylasehemmer mit einer Hemmwirksamkeit gegen menschliche Pankreasamylase von weniger als 0,1 der Hemmwirksamkeit gegen menschliche Speichelamylase, der aufweist:

(a) ein Molekulargewicht im Bereich von 23.000 bis 24.000, bestimmt durch Gelfiltration, (b) ein Ultraviolett-Absorptionsspektrum, wie es in Fig. 1 der anliegenden Zeichnungen für eine 0,22 gewichtsprozentige wässerige Lösung des Amylasehemmers gezeigt ist, (c) ein Infrarot-Absorptionsspektrum, wie es in Fig. 2 der anliegenden Zeichnungen im Falle der Tablettierung mit Kaliumbromid gezeigt ist, (d) einen isoelektrischen Punkt von 5,0, (e) einen Kohlenhydratgehalt von Null und (f) die folgende Aminosäurenzusammensetzung:

| | | | |
|-----|-------------|------------|-------------|
| Tyr | 17 Einheiten | Ser | 12 Einheiten |
| Leu | 6 Einheiten | Thr | 6 Einheiten |
| Ile | 4 Einheiten | Asp | 17 Einheiten |
| Met | 10 Einheiten | Arg | 9 Einheiten |
| Val | 24 Einheiten | His | 2 Einheiten |
| Ala | 24 Einheiten | Lys | 9 Einheiten |
| Gly | 22 Einheiten | Phe | 5 Einheiten |
| Pro | 11 Einheiten | 1/2 Cys | 14 Einheiten |
| Glu | 22 Einheiten | | |

2. Verfahren zur Herstellung des Amylasehemmers nach Anspruch 1, welches umfaßt das Einweichen von Hart- oder Durumweizen in Wasser oder einem Wasser/organischen Lösungsmittelgemisch, das Behandeln von dem so erhaltenen Extrakt durch eine Farbstoff-Liganden- Affinitäts- Chromatographie, das Eluieren des Amylasehemmers von dem verwendeten immobilisierten Farbstoff und das Isolieren des Hemmers aus dem Eluat.

3. Verfahren nach Anspruch 2, wobei der verwendete Farbstoff ein Cyanurchlorid- Derivat ist.

4. Verfahren nach Anspruch 3, wobei der Farbstoff Reaktivrot oder Reaktivblau ist.

5. Verfahren nach einem jeden der Ansprüche 2 bis 4, wobei der Hemmer durch Wiederholung der Farbstoff- Liganden- Affinitäts- Chromatographie oder durch Gelfiltration, fraktionierte Fällung unter Verwendung von Alkohol oder Ammoniumsulfat oder Dialyse gereinigt ist.

6. Verfahren zur quantitativen Bestimmung des Gehalts an Amylaseisoenzym in einer Flüssigkeit, welches das Hemmen der Speichelamylase- Wirksamkeit in der Flüssigkeit durch Zusatz des Amylasehemmers nach Anspruch 1 und die anschliessende Bestimmung des Amylaseisoenzyms umfaßt.

**Revendications**

1. Inhibiteur d'amylase ayant une activité inhibitrice sur l'amylase pancréatique humaine inférieure au dixième de son activité inhibitrice sur l'amylase salivaire humaine, cet inhibiteur ayant:

(a) un poids moléculaire de 23.000 à 24.000 déterminé par filtration sur gel;

(b) un spectre d'absorption dans l'ultraviolet tel que représenté sur la figure 1 des dessins annexés, pour une solution aqueuse à 0,22% en poids de l'inhibiteur d'amylase;

(c) un spectre d'absorption infrarouge, en pastilles de bromure de potassium, tel que représenté sur la figure 2 des dessins annexés;

(d) un point isoélectrique de 5,0;

(e) une teneur en hydrate de carbone nulle; et

(f) la composition en acides aminés suivante:

| | | | |
|-----|-----------|---------|-----------|
| Tyr | 17 unités | Ser | 12 unités |
| Leu | 6 unités | Thr | 6 unités |
| Ile | 4 unités | Asp | 17 unités |
| Met | 10 unités | Arg | 9 unités |
| Val | 24 unités | His | 2 unités |
| Ala | 24 unités | Lys | 9 unités |
| Gly | 22 unités | Phe | 5 unités |
| Pro | 11 unités | 1/2 Cys | 14 unités |
| Glu | 22 unités | | |

2. Procédé de production d'un inhibiteur d'amylase selon la revendication 1, qui comprend les étapes qui consistent à tremper du blé dur dans de l'eau ou dans un solvant aqueux/organique, à soumettre l'extrait ainsi obtenu à une chromatographie d'affinité de coordinats de colorants, à éluer l'inhibiteur d'amylase pour le séparer du colorant immobilisé employé et à isoler l'inhibiteur de l'éluat.

3. Procédé selon la revendication 2, dans lequel le colorant est un dérivé du chlorure de cyanuryle.

4. Procédé selon la revendication 3, dans lequel le colorant est du rouge réactif ou du bleu réactif.

5. Procédé selon l'une quelconque des revendications 2 à 4, dans lequel l'inhibiteur est purifié par répétition de la chromatographie d'affinité de coordinats de colorants ou par filtration sur gel, précipitation fractionnée au moyen d'un alcool ou de sulfate d'ammonium ou dialyse.

6. Procédé de dosage quantitatif de la présence d'isoenzyme d'amylase dans un fluide, qui comprend l'inhibition de l'activité de l'amylase salivaire dans le fluide par addition à celui-ci d'un inhibiteur d'amylase selon la revendication 1, puis la réalisation du dosage de l'isoenzyme d'amylase voulu.

FIG. 1

ABSORBANCE

WAVELENGTH

200                    250                    300

nm

# FIG. 2

FIG. 3

FIG. 4

FIG. 6

FIG. 5

PERCENT INHIBITION

PERCENT INHIBITION

CONCENTRATION OF INHIBITOR

CONCENTRATION OF INHIBITOR

100%

50

mg/L

FIG. 8

FIG. 7

FIG. 9

FIG. 10

r=0.99
Y=1.0x+8.0
N=20

METHOD OF THE INVENTION